(19) Europäisches Patentamt
European Patent Office
Office européen des brevets

(11) Veröffentlichungsnummer: **0 348 717**
**A2**

(12) # EUROPÄISCHE PATENTANMELDUNG

(21) Anmeldenummer: **89110526.4**

(22) Anmeldetag: **10.06.89**

(51) Int. Cl.[4]: **C07C 43/29 , C07C 49/813 , C07C 15/14 , C07C 43/225 , C07C 69/76 , C07D 333/18**

(30) Priorität: **25.06.88 DE 3821567**

(43) Veröffentlichungstag der Anmeldung:
**03.01.90 Patentblatt 90/01**

(84) Benannte Vertragsstaaten:
**DE ES FR GB IT NL**

(71) Anmelder: **BAYER AG**

**D-5090 Leverkusen 1 Bayerwerk(DE)**

(72) Erfinder: **Himmler, Thomas, Dr.**
**Bonhoefferstrasse 20**
**D-5000 Köln 80(DE)**
Erfinder: **Braden, Rudolf, Dr.**
**Nothauser Feld 1**
**D-5068 Odenthal-Scheuren(DE)**
Erfinder: **Genz, Joachim, Dr.**
**Bodelschwinghstrasse 16**
**D-4150 Krefeld(DE)**
Erfinder: **Idel, Karsten-Josef, Dr.**
**Am Schwarzkamp 38**
**D-4150 Krefeld(DE)**
Erfinder: **Pakull, Ralf, Dr.**
**Neue Linner Strasse 75**
**D-4150 Krefeld(DE)**

(54) **Lösliche Polyaromaten.**

(57) Verfahren zur Herstellung von Polyaromaten, in dem man eine aromatische Dichlorverbindung, gegebenenfalls in Anwesenheit eines wasserfreien aprotischen Lösungsmittels mit metallischem Zink, Mangan oder Magnesium umsetzt in Anwesenheit eines Katalysatorsystems aus

(1) 0,1 bis 25 Mol-% (bezogen auf Dihalogenverbindung) eines Nickelsalzes, Nickelkomplexsalzes oder Nickelkomplexes,

(2) 2 bis 100 Mol pro Mol Nickel in (1) eines Triarylphosphins,

und gegebenenfalls

(3) 0,1 bis 10 Mol pro Mol Nickel in (1) eines Alkali-, Erdalkali-, Zink-, Magnesium- oder Mangan-Halogenids.

EP 0 348 717 A2

## Lösliche Polyaromaten

Polyaromaten haben wegen ihrer thermischen Stabilität und potentiellen elektrischen Leitfähigkeit Interesse gefunden.

Die bekannteste Methode zu ihrer Herstellung ist die Polymerisation von Benzol und einigen substituierten Benzolen in Gegenwart von Lewissäuren, einem Co-Katalysator und einem Oxidationsmittel (J. Polym. Sci. Part D (Macromolecular Reviews) 5 (1971) 385 bis 430). Die Produkteigenschaften hängen stark vom verwendeten Katalysator ab.

Andere Methoden zur Herstellung von Polyarylenen sind die Wurtz-Fittig-Reaktion, in der z.B. p-Dichlorbenzol mit metallischem Natrium oder Natrium-Kalium-Legierungen umgesetzt wird, und die Ullmann-Synthese, in der Dibrom-, bevorzugt Diiodarylverbindungen durch Einwirken von metallischem Kupfer polymerisiert werden.

Eine weitere Möglichkeit besteht in der übergangsmetallkatalysierten Polymerisation der Mono-Grignard-Verbindungen von Dihalogenaromaten, z.B. der Polymerisation von p-Dibrombenzol mit Magnesium in Gegenwart von 2,2′-Bipyridyl-nickel(II)-chlorid (Japanische Patentanmeldung 52-154 900) oder der Polymerisation von 1,4-Dibromnaphthalin mit Magnesium in Gegenwart von Nickel(II)-acetylacetonat als Katalysator (Makromol. Chem. 184 (1983) 2241 bis 2249).

Dieses Verfahren erlaubt auch die Herstellung von Polyhetarylenen, wie z.B. Polythiophenen (US-PS 4 521 589).

Diese Methode ist in den meisten Fällen nur mit Dibromverbindungen als Ausgangsprodukte durchführbar; da intermediär Grignard-Verbindungen auftreten, ist auch die Möglichkeit, substituierte Ausgangsprodukte einzusetzen stark eingeschränkt. Die Methode ist also nicht universell verwendbar.

In manchen Fällen kann man das Magnesium durch metallisches Zink ersetzen. So ist die Polymerisation von 2,5-Dibromthiophen und von 1,4-Diiodbenzol mit metallischem Zink in Gegenwart von Palladium- oder Nickelkatalysatoren bekannt (Makromol. Chem., Rapid Commun. 6 (1985) 671 bis 674). Dieses Verfahren ist jedoch praktisch auf Diiod- und Dibromaromaten als Ausgangsprodukte beschränkt. Dichloraromaten kann man im allgemeinen nicht umsetzen. Es ist lediglich die Umsetzung von 4,4′-Dichlorbenzophenon mit Bis-(diphenylphosphino)-ethan-nickel(II)-chlorid bekannt, die sehr langsam abläuft und einen schwer zugänglichen Katalysator erfordert.

Es gibt also bisher keine einfache, allgemein anwendbare Methode zur Herstellung substituierter Polyarylene aus leicht zugänglichen Ausgangsstoffen.

Gegenstand der Erfindung ist ein Verfahren zur Herstellung von Polyaromaten, dadurch gekennzeichnet, daß man eine aromatische Dichlorverbindung, die auch substituiert sein kann, außer mit aciden Substituenten und Nitrogruppen, gegebenenfalls in Anwesenheit eines wasserfreien aprotischen Lösungsmittels mit metallischem Zink, Mangan oder Magnesium umsetzt in Anwesenheit eins Katalysatorsystems aus

(1) 0,1 bis 25 Mol-% (bezogen auf die Dihalogenverbindung) eines Nickelsalzes, Nickelkomplexsalzes oder Nickelkomplexes,

(2) 2 bis 100 Mol pro Mol Nickel in (1) eines Triarylphosphins,

und gegebenenfalls

(3) 0,1 bis 10 Mol pro Mol Nickel in (1) eines Alkali-, Erdalkali-, Zink-, Magnesium- oder Mangan-Halogenids, -phosphate oder -sulfate als Promoter bei Temperaturen von 0 bis 250 °C und den gebildeten Polyaromaten isoliert.

Bevorzugt ist das Verfahren zur Herstellung von Polyaromaten mit der wiederkehrenden Struktureinheit

$$-\left[ A \right]_n- \qquad (I),$$

worin n eine Zahl von 3 bis 1000 ist und A für einen aromatischen Rest der Formel

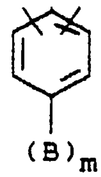

(II),

oder einen Rest der Formel

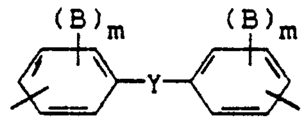

(III),

wobei in den aromatischen Ringen der Formeln (II) und (III) auch jeweils ein bis drei CH-Einheiten durch Stickstoffatome ersetzt sein können,
mit Y = CO;
COO;
$CONR^1$ mit $R^1$ = Wasserstoff, $C_1$- bis $C_6$-Alkyl, $C_6$- bis $C_{10}$-Aryl;
SO;
$SO_2$;

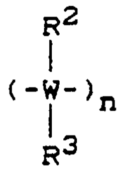

mit n = 1 bis 6, W = C oder Si und $R^2$, $R^3$ unabhängig voneinander gleich oder verschieden Wasserstoff, $C_1$- bis $C_6$-Alkyl (gegebenenfalls ein- oder mehrfach substituiert durch Halogen, bevorzugt Fluor), $C_6$- bis $C_{10}$-Aryl (gegebenenfalls ein- oder mehrfach substituiert durch Halogen, bevorzugt Fluor), Halogen, bevorzugt Fluor;
Sauerstoff;
$NR^4$ mit $R^4$ gleich Wasserstoff, $C_1$-$C_6$-Alkyl, $C_6$-$C_{10}$-Aryl
oder einen heterocyclischen Rest der Formel

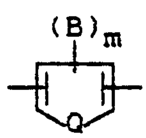

(IV)

steht
mit B = Wasserstoff;

$C_1$-$C_{18}$-Alkyl
$C_6$-$C_{14}$-Aryl
{ gegebenenfalls ein- oder mehrfach substituiert durch Halogen, (bevorzugt Fluor), Alkoxy, Cyano, Dialkylamino;

$C_1$-$C_6$-Alkoxy;
Fluoralkyl mit oder 2 C-Atomen und 1 bis 5 Fluoratomen;

$COO-C_1-C_{18}$-Alkyl; und -cycloalkyl; $COO-C_6-C_{10}$-Aryl; } gegebenenfalls ein oder mehrfach substituiert durch Halogen, (bevorzugt Fluor), Alkoxy, Cyano, Dialkylamino

Fluoralkoxy mit 1 oder 2 C-Atomen und 1 bis 5 Fluoratomen;
Phenoxy, gegebenenfalls substituiert;
$C_2-C_{16}$-Dialkylamino;
$C_6-C_{12}$-Diarylamino;
Diacetylamino;
Formyl;
Cyano;

$-CO-C_1-C_6$-Alkyl; $-CO-C_6-C_{10}$-Aryl; $-OCO-C_1-C_{18}$-Alkyl; $-OCO-C_6-C_{10}$-Aryl; } gegebenenfalls ein- oder mehrfach substituiert durch Halogen, (bevorzugt Fluor)

$-CONR^5R^6$ mit $R^5$ und $R^6$ unabhängig voneinander gleich oder verschieden $C_1-C_{18}$-Alkyl und $C_6-C_{10}$-Aryl;

$-C_1-C_{18}$-Alkylsulfinyl; $-C_6-C_{10}$-Arylsulfinyl; $-C_1-C_{18}$-Alkylsulfonyl; $-C_6-C_{10}$-Arylsulfonyl; } gegebenenfalls ein- oder mehrfach substituiert durch Halogen, (bevorzugt Fluor)

Q = S oder NR mit R = $C_1-C_{18}$-Alkyl, Benzyl, Phenyl (gegebenenfalls substituiert), Formyl, Acetyl,
m = 1 oder 2
wobei im Falle m = 2
beide Reste B zusammen auch

$-CH=CH-CH=CH-$; $-CH=CH-CH_2-$; $-CH_2CH_2CH_2-$; $-CH_2CH_2CH_2CH_2-$; $-OCH_2O-$; $-OCH_2CH_2O-$; } gegebenenfalls ein- oder mehrfach substituiert durch Halogen, bevorzugt Fluor

bedeuten können,
das dadurch gekennzeichnet, daß man eine Dichlorverbindung der Formel
Cl-A-Cl (V)
worin A die oben angegebene Bedeutung hat, gegebenenfalls in Anwesenheit eines wasserfreien aprotischen Lösungsmittels, mit metallischem Zink, Mangan oder Magnesium umsetzt in Anwesenheit eines

Katalysatorsystems aus

(1) 0,1 bis 25 Mol-% (bezogen auf Dihalogenverbindung) eines Nickelsalzes, Nickelkomplexsalzes oder Nickelkomplexes, (2) 2 bis 100 Mol pro Mol Nickel in (1) eines Triarylphosphins, und gegebenenfalls

(3) 0,1 bis 10 Mol pro Mol Nickel in (1) eines Alkali-, Erdalkali-, Zink-, Magnesium- oder Mangan-Halogenids, -phosphats oder -sulfats, bei Temperaturen von 0 bis 250 °C und den gebildeten Polyaromaten isoliert.

Ein weiterer Gegenstand der Erfindung sind Polyaromaten der Formel (I), worin n eine Zahl von 3 bis 1000 ist und A für eine aromatischen Rest der Formel

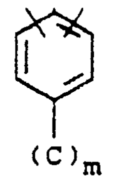

(VII)

oder einen Rest der Formel

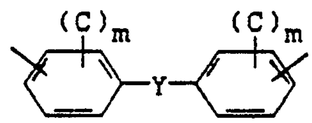

(VIII),

wobei in den aromatischen Ringen der Formeln (VII) und (VIII) auch jeweils ein bis drei CH-Einheiten durch Stickstoffatome ersetzt sein können,
mit Y = CO;
COO;
$CONR^1$ mit $R^1$ = Wasserstoff, $C_1$-$C_6$-Alkyl, $C_6$-$C_{10}$-Aryl;
SO;
$SO_2$;

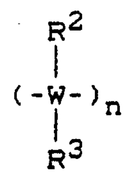

mit n = 1 bis 6, W = C oder Si und $R^2$, $R^3$ unabhängig voneinander gleich oder verschieden Wasserstoff, $C_1$-$C_6$-Alkyl (gegebenenfalls ein- oder mehr-fach substituiert durch Halogen, bevorzugt Fluor), $C_6$-$C_{10}$-Aryl (gegebenenfalls ein- oder mehrfach substituiert durch Halogen, bevorzugt Fluor), Halogen, (bevorzugt Fluor);
Sauerstoff;
$NR^4$ mit $R^4$ = Wasserstoff, $C_1$-$C_6$-Alkyl, $C_6$-$C_{10}$-Aryl

mit C = COO-$C_1$-$C_{18}$-Alkyl und -Cycloalkyl; COO-$C_6$-$C_{10}$-Aryl; } gegebenenfalls ein- oder mehrfach substituiert durch Halogen, (bevorzugt Fluor),

Phenoxy, gegebenenfalls substituiert;
$C_2$-$C_{16}$-Dialkylamino;
$C_6$-$C_{12}$-Diarylamino;
Diacetylamino;
Formyl;

Cyano;

$-CO-C_1-C_6$-Alkyl; $-CO-C_6-C_{10}$-Aryl; $-OCO-C_1-C_{18}$-Alkyl; $-OCO-C_6-C_{10}$-Aryl; gegebenenfalls ein- oder mehrfach substituiert durch Halogen, (bevorzugt Fluor)

$-CONR^5R^6$ mit $R^5$ und $R^6$ unabhängig voneinander gleich oder verschieden $C_1$-$C_{18}$-Alkyl und $C_6$-$C_{10}$-Aryl

$-C_1-C_{18}$-Alkylsulfinyl; $-C_6-C_{10}$-Arylsulfinyl; $-C_1-C_{18}$-Alkylsulfonyl; $-C_6-C_{10}$-Arylsulfonyl; gegebenenfalls ein- oder mehrfach substituiert durch Halogen, (bevorzugt Fluor),

m = 1 oder 2,
wobei im Falle von m = 2 beide Reste C zusammen auch

$-OCH_2O-$; $-OCH_2CH_2O-$; gegebenenfalls ein- oder mehrfach substituiert durch Halogen, (bevorzugt Fluor)

bedeuten können.

Das in dem erfindungsgemäßen Verfahren zur Anwendung kommende Katalysatorsystem enthält eine vorzugsweise wasserfreie Nickelverbindung, einen Liganden aus der Gruppe der Triarylphosphine mit 6 bis 10 C-Atomen in jeder Aryleinheit, gegebenenfalls einen Promotor aus der Gruppe der Alkali-, Erdalkali-, Magnesium-, Mangan- und Zinkhalogenide, -sulfate oder -phosphate und ein Metall wie Zink, Mangan oder Magnesium. Gegebenenfalls kann als weitere Komponente eine polycyclische aromatische Verbindung mit wenigstens zwei Stickstoffatomen in verschiedenen aromatischen Ringen und 10 bis 18 Kohlenstoffatomen zugesetzt werden.

Geeignete wasserfreie Nickelverbindungen sind solche, die durch die genannten Metalle reduziert werden können oder bereits in einer solchen reduzierten Form vorliegen. Ohne Anspruch auf Vollständigkeit seien hier genannt: Nickel(II)-halogenide wie Nickel(II)-chlorid, -bromid und iodid, Nickel(II)-sulfat, Nickel(II)-carbonat, Nickel(II)-salze organischer Säuren mit 1 bis 18 Kohlenstoffatomen, Nickel(II)-Komplexe wie Nickelacetylacetonat und Dichloro-bis(triphenylphosphin)-nickel(II) und Nickel(O)-Komplexe wie Bis-(cycloocta-1,5-dien)-nickel(O) oder Tetrakis(triphenylphosphin)-nickel(O), die nach literaturbekannten Verfahren herstellbar sind. Bevorzugt sind die genannten Nickel(II)-halogenide, besonders bevorzugt ist Nickel(II)-chlorid, das sich in bekannter Weise, z.B. aus dem Hexahydrat mit Thionylchlorid in wasserfreier Form erhalten läßt.

Der Nickelkatalysator wird in einer Menge von 0,1 bis 25 Mol-%, bezogen auf die eingesetzte Dichloraryl-Verbindung, bevorzugt von 1 bis 20 Mol-% und besonders bevorzugt von 3 bis 15 Mol-% eingesetzt.

Geeignete Triarylphosphine sind z.B. Triphenylphosphin, die Tri-tolyl-phosphine und Tri-naphthylphosphine. Besonders bevorzugt ist Triphenylphosphin.

Das Triarylphosphin wird in einem Molverhältnis zur Nickelverbindung von 2 bis 100, bevorzugt 4 bis 50 und besonders bevorzugt 5 bis 10 eingesetzt.

Als Promotoren werden Alkali-, Erdalkali-, Zink-, Magnesium- und Manganhalogenide bevorzugt. Besonders bevorzugt sind die Iodide.

Die Menge an Promoter kann 0,1 bis 1000 Mol-%, bezogen auf eingesetzten Nickelkatalysator,

betragen; bevorzugt sind 1 bis 700 Mol-% und besonders bevorzugt 10 bis 500 Mol-%.

Als bevorzugte polycyclische aromatische Verbindungen mit wenigstens 2 Stickstoffatomen seien 2,2′-Bipyridyl und 1,10-Phenanthrolin genannt, die bevorzugt in Anteilen von 50 bis 500 Mol-%, bezogen auf eingesetzten Nickelkatalysator, zugesetzt werden.

Als Metalle können Zink, Mangan oder Magnesium verwendet werden. Bevorzugt ist dabei Zink. Das Zink wird beispielsweise als Zinkstaub eingesetzt, der vorher durch Waschen mit Eisessig, Wasser und Aceton und anschließendes Trocknen im Vakuum gereinigt werden kann.

Das verwendete Metall kann auch im Unterschuß eingesetzt werden; bevorzugt setzt man jedoch mindestens ein Grammatom Metall pro Mol Dichlorverbindung ein, besonders bevorzugt einen Überschuß von bis zu 10 Äquivalenten.

Die Reaktion kann mit oder ohne Lösungsmittel durchgeführt werden.

Geeignete Lösungsmittel für das erfindungsgemäß Verfahren sind aprotische polare Lösungsmittel, z.B. Dimethylformamid, Dimethylacetamid, N-Methyl-caprolactam, N-Methyl-pyrrolidinon, Dimethylsulfoxid, Tetramethylensulfon usw. Die Lösungsmittel werden vor Verwendung in bekannter Weise getrocknet und unter einem Schutzgas, z.B. Stickstoff, destilliert.

Die Reaktion kann bei Temperaturen zwischen 0 und 250° C durchgeführt werden, bevorzugt bei 20 bis 200° C, besonders bevorzugt bei 50 bis 150° C.

Vorteilhaft wird die Kupplungsreaktion in einer inerten Schutzgasatmosphäre wie z.B. Helium, Argon oder Stickstoff durchgeführt.

Die Reaktion wird im allgemeinen bei Normaldruck durchgeführt, kann aber auch bei erhöhtem oder vermindertem Druck ablaufen.

Die Reaktion liegt zwischen 0,05 und 14 Stunden, bevorzugt 0,1 und 10 Stunden und besonders bevorzugt 0,5 und 8 Stunden.

Die Polyarylene finden Verwendung z.B. als hochtemperaturstabile Fasern, Filme und Beschichtungen, als Modifikatoren für Kunststoffe, als flüssigkristalline Materialien und als Vorstufen für andere Polymere.

Beispiel 1 Polyphenylen

Unter einer Stickstoffatmosphäre wurden 131 g Zinkpulver, 15 g Natriumiodid, 131 g Triphenylphosphin und 6,5 g wasserfreies Nickel(II)-chlorid in 600 ml absolutiertem Dimethylformamid vorgelegt. Nach einer halben Stunde bei 50 bis 60° C resultierte eine tief rot-braune Suspension. Zu dieser wurde eine Lösung von 147 g p-Dichlorbenzol in 100 ml absolutiertem Dimethylformamid getropft. Nach 6 Stunden bei 80° C wurde das Reaktionsgemisch mit 2 l Chloroform versetzt und mit 1 l Wasser ausgeschüttelt. Die organische Phase mit suspendiertem Feststoff wurde abgetrennt, der Feststoff abfiltriert und mit verdünnter wäßriger Salzsäure verrührt. Nach Waschen des Feststoffes mit Wasser und Trocknen wurden 42 g Polyphenylen erhalten.

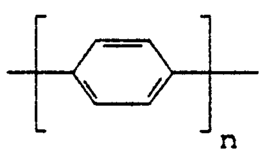 ,

Fp.: >300° C.

Die Elementaranalyse ergab einen Gehalt von:

C 86,95 %, H 5,25 %, Cl 5,6 %

Daraus ergab sich unter der Annahme, daß beide Endgruppen Chlor sind, n zu ca. 16.

Beispiel 2 Poly-(carboxymethyl)-phenylen

Unter einer Stickstoffatmosphäre wurde 157 g Zinkpulver, 262 g Triphenylphosphin, 30 g Natriumiodid und 13 g wasserfreies Nickel(II)-chlorid in 1,2 l absolutiertem Dimethylformamid vorgelegt. Nach einer halbe Stunde bei 50 bis 60° C resultierte eine tief rot-braune Suspension. Zu dieser wurde eine Lösung von 410 g 2,5-Dichlorbenzoesäure-methylester in 400 ml absolutiertem Dimethylformamid getropft. Nach 6 Stunden bei 80° C wurde das Lösungsmittel abdestilliert, der Rückstand in 5 l Chloroform gelöst und mit 3 l

verdünnter wäßriger Salzsäure gut verrührt. Nach Filtration wurde die organische Phase abgetrennt und mit 2 l Wasser ausgeschüttelt. Es wurde ca. 1 bis 2 l Chloroform abdestilliert; die verblieben e Lösung wurde mit ca. 4 l Methanol versetzt. Die resultierende Ausfällung wurde abgesaugt und getrocknet. Das so erhaltene Rohprodukt wurde zu einem feinen Pulver zermahlen und dann mit 2 bis 3 l Acetonitril für ca. 2 Stunden aufgekocht. Die Suspension wurde heiß abgesaugt und der Rückstand getrocknet. Auf diese Weise wurden 240 g Poly-(carboxymethyl)-phenylen erhalten.

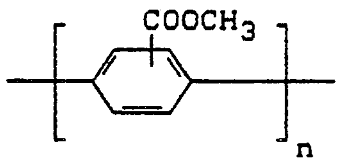 ,

Fp.: >300 °C.

Die Elementaranalyse ergab eine Gehalt von:

C 71,15 %, H 4,75 %, Cl 0,50%, 0 23,15%.

Daraus ergab sich unter der Annahme, daß beide Endgruppen Chlor sind, n zu ca. 100.

Analog Beispiele 1 und 2 wurden die in der Tabelle 1 aufgeführten Versuche durchgeführt.

Tabelle 1: Oligomerisation von Dichloraromaten (5 Mol-% Ni; $PPh_3$:Ni = 10; 200 Mol-% NaJ, bezogen auf die Ni-Verbindung, Reaktionsdauer 6 h; Reaktionstemperatur 80°C)

| Bsp. | Dichlor-Verbindung | Äquilente Zn | DMF (ml/mol) | iso. Ausbeute (%) | mittlere Kettenlänge $\bar{n}$ |
|---|---|---|---|---|---|
| 3 | 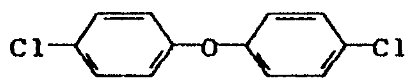 | 1,4 | 940 | 27 | 5 |
| 4 | 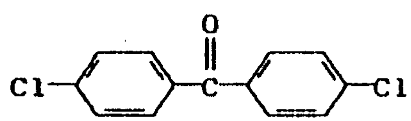 | 1,2 | 1200 | 52 | 3 |
| 5 | 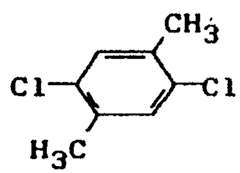 | 1,2 | 1000 | 21 | 6 |
| 6 | 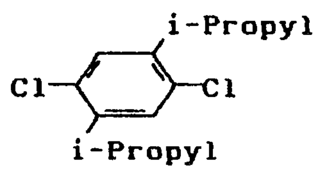 | 1,2 | 800 | 28 | 3 |

Tabelle 1: (Fortsetzung)

Oligomerisation von Dichloraromaten (5 Mol-% Ni; $PPh_3$:Ni = 10; 200Mol-% NaJ, bezogen auf dieNi-Verbindung; Reaktionsdauer 6 h; Reaktionstemperatur 80°C)

| Bsp. | Dichlor-Verbindung | Äqui-valente Zn | DMF (ml/mol) | isol. Ausbeute (%) | mittlere Kettenlänge n̄ |
|---|---|---|---|---|---|
| 7 | 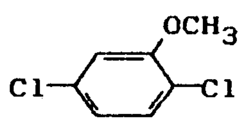 | 1,2 | 800 | 39 | 17 |
| 8 | 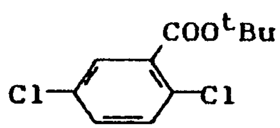 | 1,2 | 900 | 50 | 75 |
| 9 | 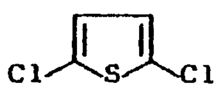 | 0,75 | 700 | 70 | 22 |

**Ansprüche**

1. Verfahren zur Herstellung von Polyaromaten, dadurch gekennzeichnet, daß man eine aromatische Dichlorverbindung, die auch substituiert sein kann, außer mit aciden Substituenten und Nitrogruppen, gegebenenfalls in Anwesenheit eines wasserfreien aprotischen Lösungsmittels, mit metallischem Zink, Mangan oder Magnesium umsetzt in Anwesenheit eines Katalysatorsystems aus
(1) 0,1 bis 25 Mol-% (bezogen auf Dihalogenverbindung) eines Nickelsalzes, Nickelkomplexsalzes oder Nickelkomplexes,
(2) 2 bis 100 Mol pro Mol Nickel in (1) eines Triarylphosphins,
und gegebenenfalls
(3) 0,1 bis 10 Mol pro Mol Nickel in (1) eines Alkali-, Erdalkali-, Zink-, Magnesium- oder Mangan-Halogenids, -phosphats ode sulfats,
bei Temperaturen von 0 bis 250 °C und den gebildeten Polyaromaten isoliert.

2. Verfahren zur Herstellung von Polyaromaten mit der wiederkehrenden Struktureinheit

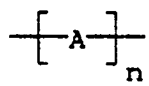 (I),

worin n eine Zahl von 3 bis 1000 ist und A für einen aromatischen Rest der Formel

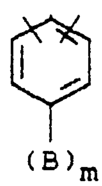 (II),

oder einen Rest der Formel

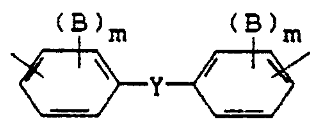 (III),

wobei in den aromatischen Ringen der Formeln (II) und (III) auch jeweils ein bis drei CH-Einheiten durch Stickstoffatome ersetzt sein können,
mit Y = CO;
COO;
$CONR^1$ mit $R^1$ = Wasserstoff, $C_1$-$C_6$-Alkyl oder $C_6$-$C_{10}$-Aryl;
SO;
$SO_2$;

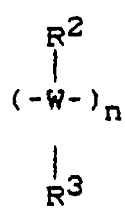

mit n = 1 bis 6, W = C oder Si und $R^2$, $R^3$ unabhängig voneinander gleich oder verschieden Wasserstoff, $C_1$- bis $C_6$-Alkyl (gegebenenfalls ein-oder mehrfach substituiert durch Halogen), $C_6$- bis $C_{10}$-Aryl, (gegebenenfalls ein-oder mehrfach substituiert durch Halogen) oder Halogen,

Sauerstoff oder
$NR^4$ mit $R^4$ gleich Wasserstoff, $C_1$-$C_6$-Alkyl, $C_6$-$C_{10}$-Aryl
oder einen heterocyclischen Rest der Formel

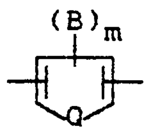

(IV)

steht
mit B = Wasserstoff;
$C_1$-$C_{18}$-Alkyl; gegebenenfalls ein- oder
$C_6$-$C_{14}$-Aryl; mehrfach substituiert durch Halogen,
Alkoxy, Cyano, Dialkylamino
$C_1$-$C_6$-Alkoxy;
Fluoralkyl mit 1 oder 2 C-Atomen und 1 bis 5 Fluoratomen;

| | |
|---|---|
| COO-$C_1$-$C_{18}$-Alkyl<br>und -Cycloalkyl;<br>COO-$C_6$-$C_{10}$-Aryl; | gegebenenfalls ein- oder mehrfach substituiert durch Halogen, Alkoxy, Cyano, Dialkylamino; |

Fluoralkoxy mit 1 oder 2 C-Atomen und 1 bis 5 Fluoratomen Phenoxy (gegebenenfalls substituiert),
$C_2$-$C_{16}$-Dialkylamino;
$C_6$-$C_{12}$-Diarylamino;
Diacetylamino;
Formyl;
Cyano;

| | |
|---|---|
| -CO-$C_1$-$C_6$-Alkyl;<br>-CO-$C_6$-$C_{10}$-Aryl;<br>-OCO-$C_1$-$C_{18}$-Alkyl;<br>-OCO-$C_6$-$C_{10}$-Aryl; | gegebenenfalls ein- oder mehrfach substituiert durch Halogen, |

-$CONR^5R^6$ mit $R^5$ und $R^6$ unabhängig voneinander gleich oder verschieden $C_1$-$C_{18}$-Alkyl oder $C_6$-$C_{10}$-Aryl;

| | |
|---|---|
| -$C_1$-$C_{18}$-Alkylsulfinyl;<br>-$C_6$-$C_{10}$-Arylsulfinyl;<br>-$C_1$-$C_{18}$-Alkylsulfonyl;<br>-$C_6$-$C_{10}$-Arylsulfonyl; | gegebenenfalls ein- oder mehrfach substituiert durch Halogen, |

Q = S oder NR mit R = $C_1$-$C_{18}$-Alkyl, Benzyl, Phenyl (gegebenenfalls substituiert), Formyl oder Acetyl,
m = 1 oder 2
wobei im Falle m = 2 beide Reste B zusammen auch

$-CH=CH-CH=CH-$;
$-CH=CH-CH_2-$;
$-CH_2CH_2CH_2-$;
$-CH_2CH_2CH_2CH_2-$;
$-OCH_2O-$;
$-OCH_2CH_2O-$;

gegebenenfalls ein- oder mehrfach substituiert durch Halogen,

bedeuten können
dadurch gekennzeichnet, daß man eine Dichlorverbindung der Formel
Cl-A-Cl (V)
worin A die oben angegebene Bedeutung hat, gegebenenfalls in Anwesenheit eines wasserfreien aprotischen Lösungsmittels, mit metallischem Zink, Mangan oder Magnesium umsetzt in Anwesenheit eines Katalysatorsystems aus (1) 0,1 bis 25 Mol-% (bezogen auf Dihalogenverbindung) eines Nickelsalzes, Nickelkomplexsalzes oder Nickelkomplexes,
(2) 2 bis 100 Mol pro Mol Nickel in (1) eines Triarylphosphins,
und gegebenenfalls
(3) 0,1 bis 10 Mol pro Mol Nickel in (1) eines Alkali-, Erdalkali-, Zink-, Magnesium- oder Mangan-Halogenids, phosphats oder -sulfats,
bei Temperaturen von 0 bis 250 °C und den gebildeten Polyaromaten isoliert.

3. Polyaromaten der Formel (I) gemäß Patentanspruch 1, worin n eine Zahl von 3 bis 1000 ist und a für einen aromatischen Rest der Formel

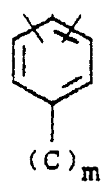

(VII),

oder einen Rest der Formel

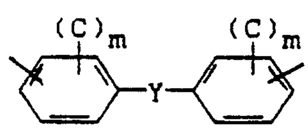

(VIII),

wobei in den aromatischen Ringen der Formeln (VII) und (VIII) auch jeweils ein bis drei CH-Einheiten durch Stickstoffatome ersetzt sein können,
mit Y = CO;
COO;
$CONR^1$ mit $R^1$ = Wasserstoff, $C_1$-$C_6$-Alkyl, $C_6$-$C_{10}$-Aryl;
SO;
$SO_2$;

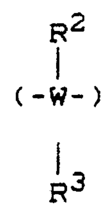

mit n = 1 bis 6, W = C oder Si und $R^2$, $R^3$ unabhängig voneinander gleich oder verschieden Wasserstoff,

$C_1$-$C_6$-Alkyl (gegebenenfalls ein- oder mehrfach substituiert durch Halogen), $C_6$-$C_{10}$-Aryl, (gegebenenfalls ein-oder mehrfach substituiert durch Halogen), Halogen;
Sauerstoff;
$NR^4$ mit $R^4$ = Wasserstoff, $C_1$-$C_6$-Alkyl, $C_6$-$C_{10}$-Aryl

mit C = COO-$C_1$-$C_{18}$-Alkyl und -Cycloalkyl; COO-$C_6$-$C_{10}$-Aryl; } gegebenenfalls ein- oder mehrfach substituiert durch Halogen

Phenoxy, gegebenenfalls substituiert;
$C_2$-$C_{16}$-Dialkylamino;
$C_6$-$C_{12}$-Diarylamino;
Diacetylamino;
Formyl;
Cyano;

-CO-$C_1$-$C_6$-Alkyl;
-CO-$C_6$-$C_{10}$-Aryl;
-OCO$C_1$-$C_{18}$-Alkyl;
-OCO$C_6$-$C_{10}$-Aryl; } gegebenenfalls ein- oder mehrfach substituiert durch Halogen,

-$CONR^5R^6$ mit $R^5$ und $R^6$ unabhängig voneinander gleich oder verschieden $C_1$-$C_{18}$-Alkyl und $C_6$-$C_{10}$-Aryl;

-$C_1$-$C_{18}$-Alkylsulfinyl;
-$C_6$-$C_{10}$-Arylsulfinyl;
-$C_1$-$C_{18}$-Alkylsulfonyl;
-$C_6$-$C_{10}$-Arylsulfonyl; } gegebenenfalls ein- oder mehrfach substituiert durch Halogen,

m = 1 oder 2,
wobei im Falle von m = 2 beide Reste C zusammen auch

-$OCH_2O$-;
-$OCH_2CH_2O$-; } gegebenenfalls ein- oder mehrfach substituiert durch Halogen,

bedeuten können.